Europäisches Patentamt

European Patent Office

Office européen des brevets

(19)

(11) Veröffentlichungsnummer: **0 153 957**
**B1**

(12) **EUROPÄISCHE PATENTSCHRIFT**

(45) Veröffentlichungstag der Patentschrift:
**27.01.88**

(21) Anmeldenummer: **84903428.5**

(22) Anmeldetag: **31.08.84**

(86) Internationale Anmeldenummer:
**PCT/DE 84/00179**

(87) Internationale Veröffentlichungsnummer:
**WO 85/00966 (14.03.85** Gazette **85/7)**

(51) Int. Cl.⁴: **A 61 F 9/00,** A 61 B 3/10,
A 61 B 3/12, G 02 B 23/08

(54) VORRICHTUNG ZUM EINKOPPELN VON OPERATIONSLICHT IN EIN AUGENUNTERSUCHUNGSGERÄT.

(30) Priorität: **31.08.83 DE 3331431**

(43) Veröffentlichungstag der Anmeldung:
**11.09.85 Patentblatt 85/37**

(45) Bekanntmachung des Hinweises auf die Patenterteilung:
**27.01.88 Patentblatt 88/4**

(84) Benannte Vertragsstaaten:
**AT BE CH DE FR GB LI NL SE**

(56) Entgegenhaltungen:
**WO - A - 84/01110**
**FR - A - 2 344 269**
**FR - A - 2 347 914**

(73) Patentinhaber: **Optische Werke G. Rodenstock,
Isartalstrasse 43, D-8000 München 5 (DE)**
Patentinhaber: **MEDITEC REINHARDT THYZEL GMBH,
Obere Bergstrasse 3, D-8501 Heroldsberg (DE)**

(72) Erfinder: **WILMS, Karl-Heinz, Hans Bierling-Str. 47,
D-8080 Emmering (DE)**
Erfinder: **SCHRÖDER, Eckhard, Hans Sachsstr. 9,
D-8501 Eckental (DE)**
Erfinder: **REIS, Werner, Dachauer Strasse 407,
D-8000 München 50 (DE)**

(74) Vertreter: **Neidl-Stippler, Cornelia, Dr. et al,
Anwaltskanzlei Münich, Neidl-Stippler, Schiller
Willibaldstrasse 36/38, D-8000 München 21 (DE)**

# Beschreibung

*Technisches Gebiet*

Die Erfindung bezieht sich auf eine Vorrichtung zum Einkoppeln von Operationslicht in ein Augenuntersuchungsgerät gemäss dem Oberbegriff des Patentanspruchs 1.

Derartige Vorrichtungen werden beispielsweise zur Koagulation der Netzhaut oder zur Lichtchirurgie menschlicher Augen benötigt.

*Stand der Technik*

Augenuntersuchungsgeräte müssen während des Untersuchungs-/Behandlungsvorgangs üblicherweise um eine vertikale Achse schwenkbar sein, die durch das zu untersuchende Auge geht. Zusätzlich sollten die Untersuchungsgeräte in der Höhe verstellbar sowie mittels eines Kreuztisches in x- und y-Richtung verschiebbar sein.

Diesen Verstellmöglichkeiten muss der Strahlengang des eingekoppelten Operationslaserstrahls folgen. Eine Vorrichtung zum Einkoppeln von Operationslicht, gemäss dem Oberbegriff des Patentanspruchs, 1, die die Verstellbarkeit des Augenuntersuchungsgeräts nicht beeinträchtigt, ist beispielsweise aus dem Deutschen Gebrauchsmuster 7 225 429 bekannt. In diesem Gebrauchsmuster wird vorgeschlagen, eine Spaltleuchte derart zu modifizieren, dass der Strahl eines Operations- bzw. Koagulationslasers durch die Spaltleuchte auf das menschliche Auge gerichtet wird. Hierzu wird der Operationslichtstrahl zunächst in die als Hohlachse ausgebildete Drehachse der Spaltleuchte eingeführt, oberhalb des Schwenkarms der Spaltleuchte wird der Operationslichtstrahl mittels eines Spiegels aus der Drehachse ausgelenkt und mittels eines zweiten Spiegels in den Strahlengang der Spaltleuchte eingeführt. Der Operationslichtstrahl tritt dabei durch einen durchbohrten Spiegel in das übliche Strahlführungssystem der Spaltleuchte ein und verläuft nach dem durchbohrten Spiegel parallel mit den Beleuchtungslicht der Spaltleuchte.

Diese bekannte Vorrichtung hat eine Reihe von Nachteilen:

Zum Einkoppeln des Operationslichtstrahls sind Eingriffe in das Strahlführungssystem der Spaltleuchte erforderlich, die deren Funktion erheblich beeinflussen. Trotz dieser Eingriffe begrenzt das Strahlführungssystem der Spaltleuchte die Apertur des Laserstrahls auf sehr kleine Werte.

Darüber hinaus kann es aufgrund der vielen optischen Flächen, durch die der Operationslichtstrahl in der Spaltleuchte hindurchgeht, zu «Geisterbildern» und Reflexionen des Operationslichtstrahls kommen. Damit ist es unter Umständen möglich, dass ein Teil des Operationslichtes auf einem falschen Weg in das Auge des Patienten eintritt und dort unbeabsichtigt Schädigungen hervorruft.

Ferner wird das Operationslicht zum Teil, nämlich auf dem Stück zwischen der Drehachse und der Spaltleuchte ohne Kapselung geführt.

Weiter ist bei der bekannten Vorrichtung nur vorgesehen, das Licht eines Operationslasers in den Strahlengang eines Augenuntersuchungsgeräts einzukoppeln. Bei der Verwendung beispielsweise eines im Infrarotbereich arbeitenden Neodym-YAG-Lasers als Operationslichtquelle wäre es aber von Vorteil, zusätzlich als Beobachtungslichtquelle bzw. als Ziellichtquelle einen «Ziellaser» zu verwenden, dessen Licht im sichtbaren Spektralbereich liegt.

Da bei der bekannten Vorrichtung der Operationslichtstrahl über die Spaltleuchte geführt wird, sind der Operationslichtstrahl und der Beobachtungsstrahlengang immer dann nicht koinzident, wenn ausgenutzt wird, dass die Spaltleuchte unabhängig vom Beobachtungsgerät schwenkbar ist. Da andererseits in vielen Anwendungsfällen eine Koinzidenz des Operationslichtstrahls und des Beobachtungsstrahlengangs erforderlich ist, kann deshalb, wie erfindungsgemäss erkannt worden ist, die an und für sich freie Verschwenkbarkeit der Spaltleuchte gegenüber dem Beobachtungsgerät bei der bekannten Vorrichtung nicht ausgenutzt werden.

Aus der WO-A-84/01110, die für die Bestimmungsstaaten DE, FR, GB, LU und NL einen Stand der Technik gemäss Art. 54(3) EPÜ darstellt, ist eine Vorrichtung der im Oberbegriff des Patentanspruchs 1 genannten Art bekannt, bei der die erste optische Einrichtung die koaxialen Lichtstrahlen in einen um die Drehachse schwenkbaren und drehfest mit dem Schwenkarm des Beobachtungsgeräts verbundenen Arm auslenkt, in dem bzw. an dem die zweite und dritte optische Einrichtung angebracht sind, die dritte optische Einrichtung die Lichtstrahlen in den Strahlengang des Beobachtungsgeräts einkoppelt und, wenn zusätzlich ein Ziellicht eingekoppelt werden soll, die Lichtstrahlen der Ziellichtquelle koaxial zum Operationslichtstrahl geführt sind. Bei dieser Vorrichtung sind also der Operationslichtstrahl und der Beobachtungsstrahlengang koinzident, auch wenn die Spaltleuchte unabhängig vom Beobachtungsgerät geschwenkt wird.

Eine Vorrichtung anderer Gattung, bei der das Licht eines Operationslasers und eines Beleuchtungslasers in den Strahlengang eines Augenuntersuchungsgeräts eingespiegelt wird, ist aus der US-A-3 769 963 bekannt. Bei dieser Vorrichtung wird das Licht des Operationslasers zwischen dem zu untersuchenden Auge und dem Beobachtungsgerät in den Strahlengang eingespiegelt, während das Licht des Beleuchtungslasers über einen Lichtleiter zum Auge geführt wird. Zusätzlich kann das Licht des Beleuchtungslasers in den Strahlengang des Operationslasers eingespiegelt werden, so dass der Lichtstrahl des Beleuchtungslasers auch als Ziellichtstrahl verwendet werden kann.

Diese bekannte Vorrichtung anderer Gattung hat jedoch den Nachteil, dass — um eine Höhenverstellbarkeit und Schwenkbarkeit des Augenuntersuchungsgeräts im üblichen Rahmen zu gewährleisten — das Licht der Laser über grosse Strecken mittels flexibler Glasfaser-Leiter geführt werden muss. Derartige Glasfaser-Leiter sind aber insbesondere für Licht im Infrarotbereich störanfällig. Darüber hinaus wird die Störanfälligkeit durch starke Beanspruchung ihrer Flexibilität erhöht, so dass nach Möglichkeit, insbesondere bei im Infrarotbereich arbeitenden Lasern, auf den Einsatz derartiger flexibler Lichtleiter verzichtet werden sollte.

*Darstellung der Erfindung*

Der Erfindung liegt die Aufgabe zugrunde, eine Vorrichtung zum Einkoppeln von Operationslicht in ein schwenkbares und verstellbares Augenuntersuchungsgerät zu schaffen, bei der ohne Beeinträchtigung der Verstellbarkeit und Schwenkbarkeit und ohne Einsatz von flexiblen Lichtleitern oder störenden Gliederoptiken sowie ohne Eingriff in das optische System des Augenuntersuchungsgeräts der Operationslichtstrahl sowie gegebenenfall zusätzlich ein Beobachtungs- und/oder Ziellichtstrahl in den Strahlengang des Beobachtungsgeräts eingekoppelt werden können.

Diese Aufgabe wird erfindungsgemäss dadurch gelöst, dass von einer Vorrichtung zum Einkoppeln von Operationslicht in ein Augenuntersuchungsgerät gemäss dem Oberbegriff des Patentanspruchs 1 ausgegangen und diese Vorrichtung durch die Merkmale des kennzeichnenden Teils weitergebildet wird.

Erfindungsgemäss werden der Operationslichtstrahl sowie gegebenenfalls die koaxial zum Operationslichtstrahl geführten Beobachtungs- und/oder Ziellichtstrahlen zunächst in der Drehachse des Augenuntersuchungsgeräts geführt und dann ausserhalb der Drehachse in den Strahlengang des Beobachtungsgeräts eingekoppelt. Damit bleibt die Höhenverstellbarkeit und die Schwenkbarkeit des Augenuntersuchungsgeräts erhalten. Durch die Auslenkung der koaxialen Lichtstrahlen in den Schwenkarm des Beobachtungsgeräts kann auf Eingriffe in den optischen Aufbau beispielsweise der Spaltleuchte verzichtet werden.

Damit hat die erfindungsgemässe Vorrichtung den Vorteil, dass die koaxialen Strahlen vollständig in Teilen der Halterung des Augenuntersuchungsgeräts geführt sind, und damit der Strahlengang ohne zusätzlichen baulichen Aufwand vollständig gekapselt ist.

Unter koaxialer Strahlführung wird dabei eine Strahlführung verstanden, bei der die Achsen der einzelnen Laserstrahlen zusammenfallen; nicht notwendigerweise muss einer der Laserstrahlen einen ringförmigen Querschnitt haben.

Weiterbildungen der Erfindung sind in den Unteransprüchen angegeben:

Die Ausbildung der Vorrichtung gemäss Anspruch 2 führt zu einer grossen Apertur, die für kleine Fleckgrössen des Operationslichtstrahls, in der Grössenordnung von 10 µm erforderlich ist.

Die Aufweitoptik kann dabei nach Anspruch 3 aus zwei optischen Elementen bestehen, von denen eines negative und das andere positive Brechkraft hat. Die optischen Elemente können dabei Einzellinsen sein oder mehrere Linsen umfassen.

Die in Anspruch 4 beanspruchte Dehnbarkeit der dritten optischen Einrichtung erlaubt die Einkoppelung des Strahlengangs weiterer Geräte, beispielsweise einer Photoeinrichtung in den Strahlengang des Augenuntersuchungsgeräts, so dass die erfindungsgemässe Vorrichtung universell einsetzbar ist.

Gemäss Anspruch 5 sind die erste, zweite und dritte optische Einrichtung Umlenkspiegel oder Umlenkprismen.

Durch die Ausbildung der erfindungsgemässen Vorrichtung nach einem der Ansprüche 6 bis 9 werden Geisterbilder, Reflexionen usw. aufgrund von Spiegelungen usw. wirksam vermieden.

Die ortsfeste Anordnung der Lichtquelle gemäss Anspruch 10 ist insbesondere dann vorteilhaft, wenn beispielsweise die Lichtquellen, insbesondere die in Anspruch 11 gekennzeichneten Laser gross und schwer sind, und damit nicht am Augenuntersuchungsgerät selbst angebracht werden können.

Die erfindungsgemässe Vorrichtung kann in Verbindungen mit den verschiedensten Augenuntersuchungsgeräten verwendet werden, beispielsweise in Verbindung mit einem Spaltlampengerät (Anspruch 12) oder mit einem Ophthalmoskop (Anspruch 13).

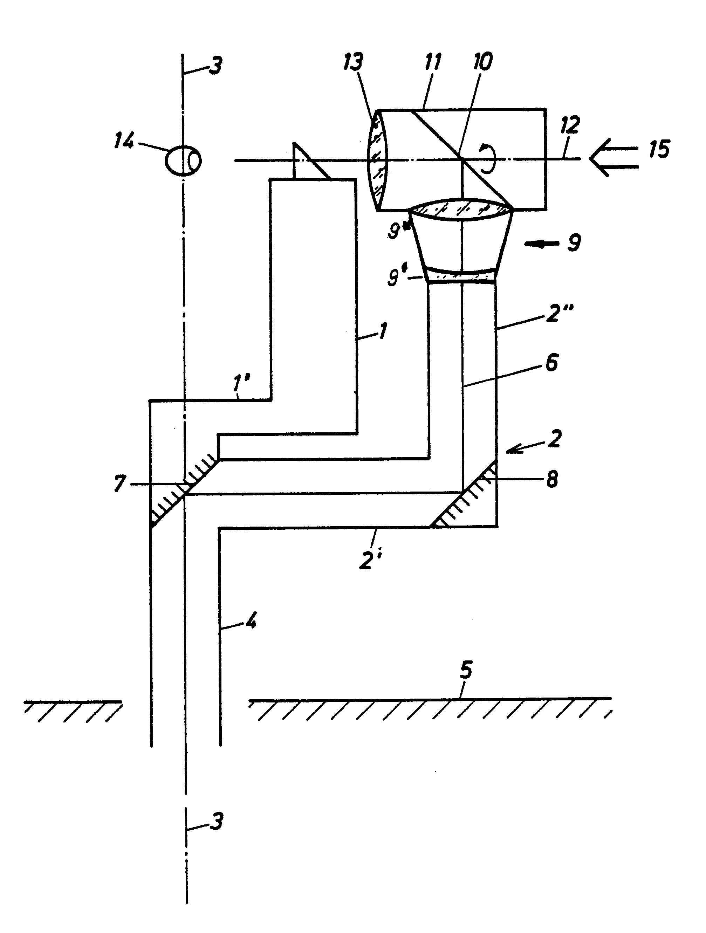

*Kurze Beschreibung der Zeichnung*

Die Erfindung wird nachstehend anhand eines Ausführungsbeispiels unter Bezugnahme auf die Zeichnung näher beschrieben, deren einzige Figur einen Querschnitt durch eine erfindungsgemässe Vorrichtung bei Verwendung an einem Spaltlampengerät zeigt.

*Weg zur Ausführung der Erfindung*

Eine nur schematisch dargestellte Spaltleuchte 1 ist mittels eines Schwenkarms 1' um eine Drehachse 3 über einen Winkelbereich von ca. ± 80° schwenkbar. An einer Halterung 4, an der der Schwenkarm 1' der Spaltleuchte 1 angebracht ist, ist zusätzlich ein Schwenkarm 2 angelenkt, an dem ein nicht näher dargestelltes Stereomikroskop 15 als Beobachtungsgerät befestigt ist.

Die Halterung 4 und damit die Spaltleuchte 1 und das Stereomikroskop sind gegenüber einem Tisch 5 in der Höhe verstellbar und zusätzlich mittels eines nicht näher dargestellten Kreuztisches in x- und y-Richtung verschiebbar.

Die Halterung 4 und der Schwenkarm 2 sind hohl, so dass in ihnen ein Lichtstrahl 6 geführt werden kann, der am unteren Ende der Halterung 4 beispielsweise mittels eines nicht näher dargestellten Spiegels oder eines Prismas eingeleitet wird. Der in die Halterung 4 eingeleitete Lichtstrahl 6 wird zunächst koaxial zur Drehachse 3 geführt und anschliessend von einem Umlenkspiegel 7 in den horizontalen Teil 2' des Schwenkarms 2 ausgelenkt. Ein Umlenkspiegel 8 lenkt den Lichtstrahl 6 in den vertikalen Teil 2'' des Schwenkarms 2 um. Der umgelenkte Lichtstrahl wird von einer Aufweitoptik 9 beispielsweise von einem Durchmesser von 6 mm auf einen Durchmesser von 30 mm aufgeweitet. Anschliessend wird der Lichtstrahl 6 von einem Umlenkspiegel 10 in einem Gehäuse 11, das am oberen Ende des senkrechten Teils 2''des Schwenkarms 2 angebracht ist, in die optische Achse 12 des Beobachtungsgeräts umgelenkt. Der aufgeweitete Strahl wird von einem Fokussierobjektiv 13, das Teil des Beobachtungsgeräts ist, auf bzw. in das zu untersuchende Auge 14 fokussiert.

Die Aufweitoptik 9 besteht aus zwei optischen Elementen 9' und 9'', von denen das Element 9' negative und das Element 9'' positive Brechkraft hat. Dabei kann jedes Element aus einer oder mehreren Linsen bestehen; das negative Element 9' kann zusätzlich beispielsweise mittels eines Revolverwechslers austauschbar ausgeführt sein.

Der Umlenkspiegel 10 kann um 360° um die optische Achse 12 gedreht werden. Hierdurch ist es möglich, auch andere optische Geräte, die an den Seiten oder an der Oberfläche des Gehäuses 11 angebracht sind und einen eigenen Strahlengang aufweisen, in den Strahlengang des Beobachtungsgeräts einzukoppeln. Ein Beispiel für ein derartiges, zusätzlich einzukoppelndes Gerät ist beispielsweise eine kleinere Lichtquelle oder ein Photozusatz.

Da die Beobachtung beispielsweise mittels des Spaltlampenmikroskops durch den teildurchlässig ausgebildeten Spiegel 10 erfolgt, können Reflexionen an den Linsen des Fokussierobjektivs 13 Geisterbilder hervorrufen.

Um derartige Geisterbilder zu vermeiden, werden unterschiedliche Bereiche des Umlenkspiegels 10 für den Zielstrahl des Helium-Neon-Lasers und den Operationsstrahl verwendet. Diese Bereichstrennung kann insbesondere dadurch erfolgen, dass die Bereiche des Umlenkspiegels, durch die die Beobachtung von der Rückseite des Gehäuses 11 her erfolgt, einem möglichst niedrigen Reflexionsfaktor für die Wellenlänge des Zielstrahls haben, dass aber die gesamte Spiegeloberfläche einen Reflexionsfaktor von nahezu 100% für die Wellenlänge des Operationsstrahls hat.

Darüber hinaus ist es vorteilhaft, die Flächen der genannten Optik, die Geisterbilder hervorrufen, reflexmindernd zu beschichten.

## Patentansprüche

1. Vorrichtung zum Einkoppeln von Operationslicht (6) in ein Augenuntersuchungsgerät, beispielsweise ein Spaltlampengerät, das eine Leuchte (1) und ein Beobachtungsgerät (15) aufweist, die um eine vertikale, durch ein zu untersuchendes Auge (14) gehende Drehachse (3) schwenkbar sind, bei der das Operationslicht (6) anfänglich in der Drehachse (3) geführt ist, eine erste optische Einrichtung (7) das Operationslicht (6) annähernd senkrecht aus der Drehachse (3) auslenkt und eine zweite optische Einrichtung (8) die Lichtstrahlen (6) annähernd parallel zur Drehachse (3) sowie eine dritte optische Einrichtung (10) die Lichtstrahlen (6) auf das Auge (14) umlenken, dadurch gekennzeichnet, dass die erste optische Einrichtung (7) die koaxialen Lichtstrahlen in den Schwenkarm (2) des Beobachtungsgeräts (15) auslenkt, in dem bzw. an dem die zweite und dritte optische Einrichtung (8 bzw. 10) angebracht sind, dass die dritte optische Einrichtung (10) die Lichtstrahlen (6) in den Strahlengang des Beobachtungsgeräts einkoppelt, und dass, wenn zusätzlich Beobachtungs- und/oder Ziellicht eingekoppelt werden soll, die Lichtstrahlen der Beobachtungs- und/oder Ziellichtquelle koaxial zum Operationslichtstrahl geführt sind.

2. Vorrichtung nach Anspruch 1, dadurch gekennzeichnet, dass eine vor der dritten optischen Einrichtung (10) angeordnete Aufweitoptik (9) die konzentrischen Lichtstrahlen (6) aufweitet, und ein nach der dritten optischen Einrichtung (10) angeordnetes Fokussierobjektiv (13) die aufgeweiteten Lichtstrahlen in das zu untersuchende bzw. zu behandelnde Auge (14) fokussiert.

3. Vorrichtung nach Anspruch 2, dadurch gekennzeichnet, dass die Aufweitoptik (9) aus einem Element (9') mit negativer Brechkraft und einem optischen Element (9'') mit positiver Brechkraft besteht.

4. Vorrichtung nach einem der Ansprüche 1 bis 3, dadurch gekennzeichnet, dass die dritte optische Einrichtung (10) zum Einkoppeln des Strahlengangs anderer Geräte in den Strahlengang (12) des Beobachtungsgeräts um mindestens 90°, vorzugsweise um 360° drehbar ist.

5. Vorrichtung nach einem der Ansprüche 1 bis 4, dadurch gekennzeichnet, dass die erste, zweite und dritte optische Einrichtung (7, 8, 10) Umlenkspiegel oder Umlenkprismen sind.

6. Vorrichtung nach einem der Ansprüche 1 bis 5, dadurch gekennzeichnet, dass zum Umlenken des Beobachtungs- und/oder Ziellichts sowie des Operationslichts getrennte Bereiche auf der dritten optischen Einrichtung (10) dienen.

7. Vorrichtung nach Anspruch 6, dadurch gekennzeichnet, dass die Bereichstrennung dadurch erzielt wird, dass der Bereich, durch den die Beobachtung erfolgt, einen möglichst geringen Reflexionsgrad für die Wellenlänge des Ziellichts und des Operationslichts hat.

8. Vorrichtung nach Anspruch 6 oder 7, dadurch gekennzeichnet, dass die gesamte Oberfläche der dritten optischen Einrichtung (10) einen Reflexionsfaktor von nahezu 100% für die Wellenlänge des Operationslichts aufweist.

9. Vorrichtung nach einem der Ansprüche 1 bis 8, dadurch gekennzeichnet, dass diejenigen optischen Flächen des Objektivs des Augenuntersuchungsgeräts reflexmindernd beschichtet sind, die störende Reflexionen des Beobachtungs- und/oder Ziellichtstrahls hervorrufen.

10. Vorrichtung nach einem der Ansprüche 1 bis 9, dadurch gekennzeichnet, dass die Lichtquellen ortsfest angeordnet sind, und Spiegel, Prismen usw. die Lichtstrahlen der Beobachtungs- und/oder Ziellichtquelle sowie der Operationslichtquelle konzentrisch in die Drehachse (3) führen.

11. Vorrichtung nach einem der Ansprüche 1 bis 10, dadurch gekennzeichnet, dass die Ziellichtquellen ein Helium-Neon-Laser und die Operationslichtquelle ein Neodym-YAG-Laser ist.

12. Vorrichtung nach einem der Ansprüche 1 bis 11, dadurch gekennzeichnet, dass das Augenuntersuchungsgerät ein Spaltlampengerät ist.

13. Vorrichtung nach einem der Ansprüche 1 bis 11, dadurch gekennzeichnet, dass das Augenuntersuchungsgerät ein Ophthalmoskop ist.

## Claims

1. Unit for coupling operational light into an eye examination apparatus such as a slot lamp equipment comprising a lamp (1) and an observation unit (15) which are both pivotable about a vertical axis of rotation (3) passing through an eye (14) under examination, wherein the operational light (6) is initially guided along the axis of rotation (3), a first optical means (7) deflects the operational light (6) approximately vertically out of the axis of rotation (3), and a second optical means (8) deflects the light beams (6) approximately in parallel to the axis of rotation (3) while a third optical means (10) deflects the light beams (6) onto the eye (14), characterized in that said first optical means (7) deflects the coaxial light beams into the swivel arm (2) of said observation unit (15) in or at which said second and third optical means (8 or 10, respectively) are provided, that said third optical means (10) couples the light beams (6) into the path of rays of the observation unit, and that whenever additional observation and/or sight-line light is to be coupled in the beams of light of the observation and/or sight-line light source are guided coaxially relative to the beam of operational light.

2. Unit according to Claim 1, characterized in that an optical expansion system (9) arranged ahead of said third optical means (10) expands the concentric beams of light (6), and that a focussing lens or objective (13) arranged downstream of said third optical means (10) focusses the expanded beams of light into the eye (14) under examination or being treated.

3. Unit according to Claim 2, characterized in that said optical expansion system (9) comprises one element (9') having a negative power of refraction and one optical element (9'') having a positive power of refraction.

4. Unit according to any of Claims 1 through 3, characterized in that said third optical means (10) for coupling the path of rays of other units into the path of rays (12) of the observation unit is suited for rotation through 90° at minimum, preferably through 360°.

5. Unit according to any of Claims 1 through 4, characterized in that said first, second and third optical means (7, 8, 10) are surface mirrors of deflecting prisms.

6. Unit according to any of Claims 1 through 5, characterized in that separate areas on said third optical means (10) are provided for deflection of the observation and/or sight-line light and of the operational light.

7. Unit according to Claim 6, characterized in that the separation of different areas is achieved by endowing the area serving for observation with the smallest degree of reflection possible for the wavelength of the sight-line light and the operational light.

8. Unit according to Claim 6 or 7, characterized in that the entire surface of the third optical means (10) has a factor of reflection in the range of nearly 100% for the wavelength of the operational light.

9. Unit according to any of Claims 1 through 8, characterized in that those optical surfaces of the objective of the ophthalmic examination unit are provided with a reflection-reducing coating, which cause the interfering reflections of the observation and/or sight-line light beam of light.

10. Unit according to any of Claims 1 through 9, characterized in that the light sources are stationarily mounted, and that guide mirrors, prisms etc. guide the beams of light of the observation and/or sight-line light source as well as the operational light source concentrically into the axis of rotation (3).

11. Unit according to any of Claims 1 through 10, characterized in that the sight-line source is a helium-neon laser while the operational light source is a neodymium YAG laser.

12. Unit according to any of Claims 1 through 11, characterized in that the eye examination unit is a slit lamp device.

13. Unit according to any of Claims 1 through 11, characterized in that eye examination unit is an ophthalmoscope.

## Revendications

1. Installation pour le couplage de lumière opérationnelle (6) dans un appareil d'examen des yeux, par exemple un appareil à lampe à fente, qui est pourvu d'une lampe (1) et un dispositif d'observation (15) qui sont pivotables autour d'une axe de pivot (3) verticale qui passe par un oeil (14) à examiner, dans laquelle la lumière opérationnelle (6) est d'abord guidée par dedans ledit axe de pivot (3), et dans laquelle un premier dispositif optique (7) défléchit la lumière opérationnelle (6) presque verticalement dudit axe de pivot (3) pendant qu'un deuxième dispositif optique (8) défléchit les faisceaux lumineux (6) à peu près en parallèle à l'axe de pivot (3), et dans laquelle enfin un troisième dispositif optique (10) défléchit les faisceaux lumineux (6) vers l'oeil (14), caractérisée en ce que ledit premier dispositif optique défléchit les faisceaux lumineux coaxiaux dans le bras pivotant (2) dudit dispositif d'observation (15) dans ou à lequel le deuxième et le troisième dispositif optique (8 ou 10) sont fixés, que le troisième dispositif optique (10) couple les faisceaux lumineux (6) dans la trajectoire des rayons du dispositif d'observation, et qu'au cas où on désire de coupler de la lumière d'observation et/ou de collimation supplémentaire, les faisceaux provenant de la source lumineuse d'observation et/ou de collimation sont guidés coaxialement au faisceaux lumineux opérationnel.

2. Installation selon la revendication 1, caractérisée en ce qu'un système d'élargissement optique (9) est pourvu avant ledit troisième dispositif optique (10) pour élargir les faisceaux lumineux concentriques (6), et qu'un objectif de focalisation (13) est disposé derrière ledit troisième dispositif optique (10) pour concentrer les faisceaux lumineux élargis dans l'oeil (14) à examiner ou traiter.

3. Installation selon la revendication 2, caractérisée en ce que le système d'élargissement optique (9) est composé d'un élément (9') à réfringence négative, et d'un élément optique (9'') à réfringence positive.

4. Installation selon quelconque des revendications 1 à 3, caractérisée en ce que ledit troisième dispositif optique (10) de couplage de la trajectoire des rayons des autres appareils dans la trajectoire (12) dudit dispositif d'observation est pivotable par 90° au moins et préférablement par 360°.

5. Installation selon quelconque des revendications 1 à 4, caractérisée en ce que le premier, le deuxième et le troisième dispositif optique (7, 8, 10) sont des miroirs ou prismes déflecteurs.

6. Installation selon quelconque des revendications 1 à 5, caractérisée en ce que des zones séparées sur ledit troisième dispositif optique (10) sont pourvues pour la déviation de la lumière d'observation et/ou de collimation ainsi que de la lumière opérationnelle.

7. Installation selon la revendication 6, caractérisée en ce qu'on atteint la séparation des zones par le fait que la zone par laquelle se fait l'observation est pouvue d'un facteur de réflexion le plus petit que possible pour la longeur d'onde de la lumière de collimation et les rayons opérationnels.

8. Installation selon la revendication 6 ou 7, caractérisée en ce que l'entière surface dudit troisième dispositif optique (10) a un facteur de réflexion à peu près de 100% pour la longueur d'onde de la lumière opérationnelle.

9. Installation selon quelconque des revendications 1 à 8, caractérisée en ce que ceux surfaces optiques de l'objectif de l'appareil d'examen des yeux, qui causent des réflexions nuisibles du faisceau d'observation et/ou de collimation, sont couvertes d'une couche antireflet.

10. Installation selon quelconque des revendications 1 à 9, caractérisée en ce que les sources lumineuses sont fixées stationnairement, et que des miroirs, prismes, etc. guident les faisceaux lumineux provenant de la source lumineuse d'observation et/ou de collimation, ainsi que de la source de lumière opérationnelle concentriquement dans l'axe de pivot (3).

11. Installation selon quelconque des revendications 1 à 10, caractérisée en ce que la source lumineuse de collimation est un laser à hélium et à néon et que la source de lumière opérationnelle est un laser au néodyme du type YAG.

12. Installation selon quelconque des revendications 1 à 11, caractérisée en ce que ledit appareil d'examen des yeux est un dispositif à lampe à fente.

13. Installation selon quelconque des revendications 1 à 11, caractérisée en ce que ledit appareil d'examen des yeux est un ophthalmoscope.